(19) Europäisches Patentamt
European Patent Office
Office européen des brevets

(11) EP 0 641 437 B1

# (12) EUROPEAN PATENT SPECIFICATION

(45) Date of publication and mention
of the grant of the patent:
**18.11.1998 Bulletin 1998/47**

(21) Application number: **92919975.0**

(22) Date of filing: **25.09.1992**

(51) Int Cl.⁶: **G01N 33/50**, G01N 1/30,
C12Q 1/48

(86) International application number:
**PCT/GB92/01768**

(87) International publication number:
**WO 93/06485 (01.04.1993 Gazette 1993/09)**

(54) **DETECTION OF MALIGNANT AND PRE-MALIGNANT CONDITIONS**

NACHWEIS VON MALIGNEN UND PRÄMALIGNEN ZUSTÄNDEN

DETECTION D'AFFECTIONS MALIGNES ET PRE-MALIGNES

(84) Designated Contracting States:
**BE CH DE FR GB IE IT LI LU**

(30) Priority: **27.09.1991 GB 9120633**

(43) Date of publication of application:
**08.03.1995 Bulletin 1995/10**

(73) Proprietor: **BRUNEL UNIVERSITY**
**Middlesex, UB8 3PH (GB)**

(72) Inventors:
• **JONAS, Sonja, Karin**
**Middlesex HA1 4DS (GB)**
• **SLATER, Trevor, F.**
**Deceased (GB)**

(74) Representative: **Williams, John Francis et al**
**WILLIAMS, POWELL & ASSOCIATES**
**4 St Paul's Churchyard**
**London EC4M 8AY (GB)**

(56) References cited:
**EP-A- 0 042 482**          **GB-A- 2 198 846**

• GYNECOLOGIC ONCOLOGY vol. 4, 1976, NEW YORK, NY, US pages 125 - 132 DONALD E. ROUNDS ET AL. 'Prospects for a Personal Screening Method for Cervical Carcinoma'
• ANALYTICAL BIOCHEMISTRY vol. 28, 1969, NEW YORK US pages 192 - 205 DAVIS H. IVES, JOHN P. DURHAM AND VIRGINIA S. TUCKER 'Rapid Determination of Nucleoside Kinase and Nucleosidase Activities with Tritium-Labeled Substrates' cited in the application
• PROCEEDINGS OF THE NATIONAL ACADEMY OF SCIENCES OF USA vol. 79, July 1982, WASHINGTON US pages 4093 - 4097 KAREN F. F. SCOTT, FRANK L. MEYSKENS,JR., DIANE HADDOCK RUSSELL 'Retinoids increase transglutaminase activity and inhibit ornithine decarboxylase activity in Chinese ovary hamster cells and in melanoma cells stimulated to differenciate' cited in the application
• English translation of SU-A-1303881 & Derwent WPI AN 87-340411

**Description**

This invention relates to the detection of malignant and pre-malignant conditions of the uterine cervix and more particularly to test procedures based on examination of cell samples derived by cervical smear.

Cancer of the cervix is a world-wide problem and in some countries a major cause of death by cancer in women. In its early and pre-malignant condition, known as cervical intra-epithelial neoplasia (CIN grades 1 to 3), the treatment of the disease is safe and successful. This emphasises the importance of early and accurate diagnosis.

The standard procedure for detection of abnormal cells, malignant or pre-malignant, is the Papanicolaou test, commonly known as the Pap-test. It was introduced in the 1940s and has to date never been subjected to any major clinical trial to test its accuracy and reliability as an indicator of early lesions of the uterine cervix. However, epidemiological evidence suggests strongly that there are disturbingly high error rates associated with this test (Mitchell & Medley, The Lancet, 1991, 337, 265-267). The methodology consists of taking samples of material from the uterine cervix and smearing this onto a glass microscope slide followed by a staining procedure. The major criticisms are that the method relies on optimum sampling and experienced but subjective diagnosis by the cytologist. The test involves tedious microscopic examination carried out for long periods which introduce boredom and consequential errors. Above all, the method is not quantitative. Alternative approaches have been devised which include measurements based on DNA staining and estimation of degrees of aneupolidy but many of these methods are time-consuming and depend on sophisticated instrumentation for diagnosis, thus preventing an obstacle to automation.

It is an object of the present invention to provide a test for the medical conditions specified above which is more accurate and reliable than the existing Pap-test.

It is a further object of this invention to provide a test of the foregoing kind which enables a quantitative estimate to be made of the condition of the patient under test.

It is a still further object of this invention to provide a test of the foregoing kind which is amenable to automated procedures.

The present invention comprises a method of testing for a malignant or pre-malignant condition of the cervix by examination of a cervical smear, which comprises selecting a fraction of the smear consisting predominantly of squamous epithelial cells and examining the selected fraction to determine characteristics indicative of the said malignant or pre-malignant condition. The smear is preferably obtained by the conventional PAP test.

Various methods are available for selecting the epithelial fraction of the cells present in the original sample for the purposes of examination and test. Selection may involve physical removal of the desired fraction from unwanted cellular material but alternative methods are possible in which selection occurs, in effect, as part of the test procedure itself. Use may be made, for example, of methods of flow cytometry in procedures in which a discrete step of separating the epithelial cells may not be necessary. Flow cytometry may be used to separate cells according to size for subsequent testing. Thus, a selective reaction of the epithelial cells, or substances present therein, with an antibody or other reagent may enable these cells to be identified and examined without the need for a prior step of physical removal of contaminant material. For example cells obtained from routine smears can be diluted in saline e.g PBS and thoroughly syringed to achieve single cell suspension. This can be fixed and stained using various fluorescent antibodies which may or may not bind to different cell types. Following washing the cell suspension can be subjected to flow cytometry. By raising a fluorescent antibody to a significant cell component e.g.an enzyme the cell type can be selected for by flow cytometry. If another fluorescent antibody is raised against another component it would be possible to measure the two antibodies simultaneously in the same population of cells.

Preferably, the desired fraction of cells is separated from other material by a discrete step which precedes the testing of the cells by methods to be described more fully hereinafter. Buoyant density methods are highly suitable for separating epithelial cells for the purposes of the invention. A particularly effective method is by density gradient centrifugation. Methods of this kind are highly effective for the removal of material such as inflammatory cells, cell debris and mucus which interfere with the assessment of the abnormalities under investigation.

Discontinuous gradient centrifugation methods are highly effective in obtaining sharp bands of the various fractions whereas continuous gradient methods are less useful and are not recommended. A wide range of gradient materials is available and suitable materials may be readily selected for use according to this invention depending on their osmolarities at the concentrations required, which must not be damaging to the cells, especially the epithelial cells. The common inorganic salt gradients eg caesium chloride are less preferred to the organic materials including glycerol, sucrose, dextran, bovine serum albumin, and the proprietary materials known as Percoll (colloidal silica product of Pharmacia) and Ficoll (a copolymer of sucrose and epichlorhydrin supplied by Pharmacia), Metrizamide and Nycodenz (iodinated aromatic compound products of Nyegaard). Percoll and Ficoll give excellent results for our purposes and are highly preferred.

It has been found that the desired band of cells to be separated is, or corresponds to, the fraction of density range from about 1.035 to about 1.055 grams per millilitre (g/ml) as measured in a Percoll density gradient. Methods other than density gradient techniques can alternatively be used to separate a fraction correlating with that in the specified Percoll density range.

After separation of the desired fraction a variety of methods of examining the cells may be utilised. Thus, the proportion of cells in the separated fraction which have abnormal characteristics can be determined by cell-counting. However, it will be understood that an important objective of the present invention is to provide a quantitative estimate of the stage to which the disease may have progressed. Quantitative methods for use according to the present invention fall into two main categories. The first of these includes tests carried out on intact cells which, for convenience of description, will be referred to herein as cytochemical methods. These methods usually involve the estimation of a marker substance formed in or taken up by the whole cells as, for example, when typical cell staining techniques are used. Cytochemical methods may make use of biochemical reactions carried out in the intact cells which result in the formation of a product which can be measured by spectrophotometric or colorimetric techniques or by other means, for example, using microdensitometry and flow cytometry.

The second category of quantitative methods for use in accordance with this invention includes tests carried out on lysed cells and cell extracts. These methods, which will be referred to as biochemical methods, are highly preferred. Biochemical methods entail the monitoring of the biochemistry of epithelial cells in order to detect differences between normal and abnormal cells. Of especial value in this connection are methods for determining the content of certain enzymes or other proteins, the expression of which may be raised above normal values in conditions of cell proliferation i.e. raised in activity or in amount, or both. For this purpose, use may be made of biochemical or immunoassay methods, including fluorescent monoclonal or polyclonal antibody binding of enzymes or other proteins. Suitable examples of such enzymes are the pentose phosphate shunt enzymes, ornithine decarboxylase (ODC), thymidine kinase (TK), and ribonucleotide reductase (RNR). At present the standard methods for the assay of both ODC and TK require the use of radioactive materials. They are described by Scott et al (1982) PNAS (USA), 79 4093 for ODC and Ives et al (1969) Anal. Biochem. 28 192 for TK. The use of non-radioactive materials in alternative methods eg colorimetric or fluorimetric methods or by antibody raising would be much preferred. At present the preferred choice is one or more of the pentose phosphate shunt enzymes, espcially glucose-6-phosphate dehydrogenase and/or 6-phosphogluconate dehydrogenase. In order to carry out such dehydrogenase assays the cells may be lysed in detergent and the extract used for the assay, for example employing substrates consisting of NADP+ and glucose-6-phosphate or 6-phosphogluconate. The oxidations may be coupled to a cycling electron acceptor eg phenazine methosulphate (PMS) and a final electron acceptor eg dichlorophenoindophenol (DcPIP) or nitroblue tetrazolium (NBT). Determination of activity may thus be achieved by spectrophotometry at 600nm or microdensitometry at 540nm.

Enzymatic assays of the foregoing kind may be readily carried out with the use of reagents supplied in the form of a kit in which the reagents are contained in separate containers in the customary way for biochemical assay kits. Each reagent may be separately packaged as a unit amount required for a single test or as multiple units from which aliquot amounts are dispensed when carrying out a series of such tests. For the above-mentioned reagents the substrate concentrations that are preferably used in order to avoid any reaction in the absence of these lie within 2.5 and 3.5 mM glucose-6-phosphate or 6-phospho gluconate; 0.45 and 0.55 mM NADP+; 0.055 and 0.065 mM DcPIP or NBT; 0.10 and 0.18 mM PMS for both the cytochemical and biochemical assay.

For the convenience of the operator, in addition to the assay reagents the kits preferably contain the gradient materials required for the preliminary centrifugal separation of the epithelial cells. These are supplied in the form of solutions or as dry materials for reconstitution at the desired concentrations over the density range necessary for the discontinuous gradient method eg as described above for Percoll.

It will therefore be appreciated that this invention provides a simple and reliable test of pre-malignancy which is based on (i) the separation of epithelial cells from the uterine cervix from contaminating material which may interfere with the test, and (ii) the development of sensitive methods for detection of abnormalities in these separated cells.

Also in accordance with this invention the sensitivity of the biochemical method can be amplified by means of an enzyme ratio measurement. Thus the assays of those enzymes mentioned above can be supplemented by measurement of the levels of activity of other enzymes which are repressed in malignant or pre-malignant cells. By the term "repressed" is meant present at a reduced level either in activity or in amount. The ratio of the two sets of enzymes (increased to decreased) gives a sensitive index of cellular abnormality. Examples of such repressed enzymes are catalase and xanthine oxidase which may be conveniently determined by known methods eg for catalase the production of oxygen in the presence of hydrogen peroxide.

Applying the biochemical methods decribed above, the enzymes of the pentose phosphate shunt have been measured, using an amplifying recycling technique, by spectrophotometry and catalase was estimated either by the reduction in $H_2O_2$ concentration or by oxygen generation in an oxygen electrode system.

Practical Examples of the cell separation procedure and the reaction systems studied to produce the activity ratios used for diagnostic purposes are described below.

EXAMPLE 1: Sampling and Gradient Separation

Sampling

Cervical material is obtained from well-woman's clinics from patients undergoing routine check-ups and from colposcopy clinics from patients who have been referred due to suspected abnormality. The samples are collected using either a wooden spatula, a Jordan's spatula or various types of cytobrushes, and placed immediately into universal bottles containing sterile cold phosphate-buffered saline. Processing of these samples can be delayed up to 6 hours but is preferably carried out immediately after collection although it is possible to partially process the samples and then freeze them in 5-10% DMSO and carry out any appropriate assay several days later with only a small loss of enzyme activity.

In order to demonstrate that loss of enzyme activity is minimised various storage conditions have been tested on a mammalian epithelial cell line. The results for G6PD activity are given in Table 1.

Table 1

| storage conditions | time of assay | G6PD activity |
|---|---|---|
| | days after processing | (units/min/$10^5$cells) |
| none | immediately (control) | 0.100 |
| -70ºC | 4 | 0 |
| -70ºC (+1% DMSO) | 4 | 0.190 |
| -70ºC (+10%DMSO) | 4 | 0.070 |
| $N_2$ | 4 | 0.004 |
| $N_2$ (+1% DMSO) | 4 | 0.021 |
| $N_2$ (+10%DMSO) | 4 | 0.065 |

In the above Table the cells come from an established cell line and there is no need for a density gradient separation process. In the case of clinical cervix material similar storage methods can be applied either before or after the density gradient separation. This is an important step in the procedure if the test is to be considered for practical application in a busy gynaecology clinic or colposcopy unit.

Gradient Separation

The discontinuous density gradient is obtained by preparing solutions of Percoll (<25mOs/kg $H_2O$ density of 1.130g/ml, Pharmacia) of different density and carefully layering these on top of each other avoiding any mixing and in descending order of density.

The formula used to prepare the dilutions is shown below:

$$V_0 = V \frac{P - (0.1 \times P_{10}) - 0.9}{P_O - 1}$$

where

$V_0 =$      volume of Percoll (stock)      ml
$V =$      volume of final working solution      ml
$P =$      desired density of final solution      g/ml
$P_0 =$      density of Percoll (stock)      g/ml
$P_{10} =$      density of 1.5M NaCl      = 1.058g/ml

Therefore for a final working solution (V) of 100 mls and a desired density (P) of 1.085g/ml and a density of the stock Percoll ($P_0$) of 1.130g/ml, the amount of stock Percoll to be added is 60.92 mls. The osmolarity is maintained by addition of 10 mls of 1.5M NaCl ($P_{10}$). The final volume of 100 mls is made up with distilled water.

The most suitable densities for the separation of cervical cells have been found to be 1.085 g/ml, 1.055g/ml, 1.035g/ml, 1.025g/ml. These may be altered by omitting one or two of the densities if the contamination in the starting material is minimal. Equally if the starting material has a high concentration of contaminating material then a repeated gradient separation may be necessary. Each solution is carefully layered on top of another.

The cell suspension is shaken off the brush or spatula into the PBS solution. Following gentle syringing using a 1 ml syringe with a 21G x 1 1/2" needle to disperse the cells, the whole suspension is centrifuged at 800-1000 rpm (400 x g) in a bench top centrifuge for 5 minutes. The supernatant is carefully removed to leave approximately 1 ml of suspension of cells. This is carefully layered onto the previously prepared discontinuous density gradient and centrifuged at 2000rpm (900 x g) for 10 minutes.

This produces several different bands of cells which can be removed into separate tubes. The top layer contains a mixture of bacterial, inflammatory and epithelial cells. The central bands contain predominantly epithelial cells and occasionally some basal cells and these are the cells of interest. There can be up to three of these central bands and they are morphologically indistinguishable from one another. These are usually combined. The bottom layer contains red blood cells, dead cells and cell debris. The cells are washed twice with PBS to remove the Percoll. Refinement of this method includes the further elimination of contaminating white blood cells. This often requires a further separation step, which involves either passing them through the same gradient a second time or using a Ficoll gradient to separate them (centrifugation at 400 x g for 10 minutes). In this case the white cells remain on the surface and the epithelial cells will settle to the bottom. The gradient preparation is entirely suitable for automation and several preformed discontinuous gradients can be frozen down and thawed without loss of the buoyant density properties.

EXAMPLE 2 : Cytochemical Determination of 6PGD activity in Whole Cells

An advantage of the cytochemical estimation is the confirmation that the activity of the enzymes of the pentose phosphate shunt being measured, actually derives from the separated cells. In this case the second enzyme in the pathway is measured because there is a longer incubation period involved during which time glucose-6-phosphate could be substantially metabolised by the glycolytic pathway. The cytochemical estimation therefore uses 6-phosphogluconate dehydrogenase as an estimate of the pentose phosphate shunt activity.

The material is obtained as described in Example 1 and similarly passed through a discontinuous Percoll density gradient. After cell selection and washing of the cells a sparse population of these is smeared onto prewashed slides and allowed to air-dry.

In this system the final electron acceptor is NBT instead of DcPIP in the biochemical system. NBT is a yellow coloured complex which, upon reduction forms a blue precipitate. The reaction sequences are :

(i) $\quad$ 6-phosphogluconate + NADP + $2H^+ \rightarrow$ ribulose-5- phosphate + $NADPH_2$

(ii) $\quad NADPH_2 + PMS \rightarrow PMSH_2 + NADP$

(iii) $\quad PMSH_2 + NBT \longrightarrow PMS +$ reduced formazan
(yellow) $\qquad$ (blue ppt)
O.D. max. 540nm)

This formazan precipitate has a characteristic absorption maximum at 540nm and is visible by light microscopy as insoluble particles within the cytoplasm of the cells. The intensity of staining is proportional to the enzyme activity and varies in each individual cell. In the absence of either 6PG or $NADP^+$ no staining occurs. The absorbance at 540 nm is measured using computerised microdensitometry. In brief, the method employs comparison of the digitized image of cells selected by the operator with a blank background field from the same slide and calculates the individual absorbances of the picture elements (pixels) from which the total and mean absorbances can be obtained. The video images are obtained using a Hitachi KP4 video camera mounted in a Zeiss photomicroscope using a narrowband interference filter ($\lambda$ =540 ± 10nm) (Glen Spectra Ltd) and a 10x objective lens. Luminosity data are handled by an Intellect 200 Image Analysing system interfaced to a PDP 11/23+ host computer employing a version of the "CYTABS" (copyright DJS) programme.

| Kit Formulation | | |
|---|---|---|
| For 100 tests: | | |
| Percoll reagents, as required glycyl glycine buffer (pH 8.5) | 60 mls of 0.5M aqueous solution (4°C) | A |
| 6-phosphogluconate (6PG.trisodium salt) | 20 mls of 10mM aqueous frozen vial (-20°C) | B |
| NADP+(sodium salt) | 10 mls of 5mM aqueous frozen vial (-20°C) | C |
| nitrotetrazolium blue (NBT) | 10 mls of 1% aqueous frozen vial (-20°C) | D |
| phenazine methosulphate (PMS) | 4.3 mgs frozen vial (-20°C) | E |

The procedure is as follows:

1. Smear the separated cells onto a clean glass slide and allow to air-dry.

2. Mix A + B + C + D + E + 10 mls of distilled water into a light protected vessel.

3. Very carefully add lml per sample onto the slide covering the entire cell smear.

4. Incubate this immediately at 37°C in the dark for 40 minutes.

5. Gently pour off the reaction mixture, wash very carefully twice with distilled water.

6. Fix in ethanol (1 minute, 70%; 1 minute 90%; 1 minute 95% and 1 minute 100%).

7. Clear in Xylene.

8. Mount in Depex mounting medium.

In normal smears the majority of cells have low absorbance values. In contrast, many of the abnormal smears, i.e. CIN 1,2 or 3 contain a proportion of cells with significantly higher absorbance values. By taking a particular "cut-off" point of 0.15 optical density units and counting the number of cells with staining intensities greater than this value it is possible to compare results from normal and precancerous samples. Adjustment of the programme to include this as an automated step is readily achievable. An example of some of the material analysed in this way is given below:

Table 2

| diagnosis | total no. cells | no. cells with O.D.540 >0.15 | percentage of total nos. of cells |
|---|---|---|---|
| normal | 119 | 0 | 0 |
| normal | 105 | 0 | 0 |
| normal | 104 | 0 | 0 |
| normal | 116 | 3 | 2.6 |
| normal | 97 | 3 | 3 |
| normal | 127 | 5 | 3.9 |
| normal | 101 | 4 | 4 |
| normal | 124 | 11 | 8.9 |
| normal | 112 | 11 | 9.8 |
| normal | 104 | 19 | 18.3 |
| CIN 1 | 114 | 1 | 0.9 |
| CIN 1 | 105 | 9 | 8.6 |
| CIN 1 | 102 | 13 | 12.7 |
| CIN1 | 107 | 17 | 15.9 |
| CIN1 | 70 | 11 | 16.0 |
| CIN 1 | 104 | 17 | 16.4 |
| CIN 1 | 102 | 23 | 23.0 |
| CIN 2 | 103 | 6 | 5.8 |

Table 2   (continued)

| diagnosis | total no. cells | no. cells with O.D.540 >0.15 | percentage of total nos. of cells |
|-----------|-----------------|------------------------------|-----------------------------------|
| CIN 2 | 66 | 8 | 12.0 |
| CIN 2 | 105 | 15 | 14.3 |
| CIN 2 | 100 | 12 | 12.0 |
| CIN 2 | 116 | 21 | 18.0 |
| CIN 2 | 111 | 28 | 25.0 |
| CIN 2 | 105 | 30 | 29.0 |
| CIN 2 | 116 | 38 | 33.0 |
| CIN 3 | 10 | 20 | 19.6 |
| CIN 3 | 116 | 23 | 19.8 |
| CIN 3 | 104 | 29 | 27.9 |
| CIN3 | 131 | 41 | 31.3 |
| CIN 3 | 120 | 40 | 33.4 |
| CIN 3 | 101 | 35 | 35.0 |
| CIN 3 | 113 | 41 | 6.3 |
| CIN 3 | 118 | 45 | 38.1 |
| CIN 3 | 112 | 63 | 56.3 |

Expressed as a percentage of the total number of cells measured, this produces a similar distribution to that of the biochemical test (see Example 3) . Taking into account that nearly $10^3$ x as many cells are measured in the biochemical assay, (see Example 3) the cytochemical results produce a greater overlap between normal and CIN samples. With advancement of the microdensitometry programmes developed for the cytochemical test a larger cell population can be measured in a shorter space of time thus increasing the sensitivity of this method to that of the biochemical test.

EXAMPLE 3 : Biochemical Determination of G6PD activity in Lysed Epithelial Cells

(1) Counting and subsequent lysis of cells

The procedure requires small tubes containing 0.4 mls PBS, 50µl 2% trypan blue and 50µl cell suspension. The separated cells are counted by haemocytometer, the trypan blue uptake noted, and the number of white cells present is estimated. The cells are centrifuged again and the PBS replaced by 0.5mls of 0.1% Nonidet P40 detergent to lyse the cells. The cell lysate is vortexed and placed on ice.

(2) Biochemical method

The method employed is based on the first step in the pentose phosphate shunt pathway, which is a salvage pathway of glucose metabolism generating NADPH necessary for biosynthesis of lipids and other reducing reactions as well as ribose-5-phosphate which is an essential precursor for the synthesis of nucleic acids. The pathway is stimulated in cell proliferation due to the increased requirement of DNA synthesis.

The assay uses two substrates (G6P and NADP$^+$), a cycling electron acceptor and a final electron acceptor. The final electron acceptor is a coloured complex, which, upon reduction loses its colour. The rate of disappearance of colour is proportional to the enzyme activity in the sample tested. The two electron acceptors are phenazine methosulphate (PMS) and 2,6-dichlorophenoindophenol (DcPIP) and the reaction is as follows:

(i)      glucose-6-phosphate $+$ NADP $+$ 2H$^+$ $\rightarrow$ 6-phosphogluconate $+$ NADPH$_2$

(ii)      NADPH$_2$ $+$ PMS $\rightarrow$ NADP $+$ PMSH$_2$

$$\text{(iii)} \quad PMSH_2 \quad + \quad DcPIP \longrightarrow PMS \quad + \quad leuco\text{-}DcPIP$$

**Blue O.D. max. 600nm)   (colourless)**

The disappearance of the blue colour is monitored by spectrophotometry at 600nm. A typical procedure consisting of cell processing and enzyme determination includes cell harvesting, density gradient centrifugation followed by counting the cells, lysing of the cells and spectrophotometric measurement.

| Kit Formulation | | |
| --- | --- | --- |
| For 20 tests | | |
| Glycyl glycine buffer | 20 mls of 0.5M (ph 8.5), 4°C | A |
| Glucose-6-phosphate (G6P, sodium salt) | 6 mls of 59 mM aqueous frozen vial,-20°C. | B |
| NADP$^+$ (sodium salt) | 5 mls of 10 mM aqueous frozen vial, -20°C. | C |
| Dichlorophenoindophenol (DcPIP) | 2.8 mls of 2 mM aqueous frozen vial, -20°C. (stored in dark vessel). | D |
| Phenazine methosulphate (PMS) | 12 mgs.in a dark vessel | E |
| Nonidet P40 | 100 mls of 0.1% (4°C) | |
| Glucose-6-phosphate dehydrogenase enzyme standard | 50 mls in 0.1% Nonidet P40(4°C) | |

In addition to the above, 4 bottles of prepared Percoll densities (1.085, 1.055, 1.035 and 1.025 g/ml) each of 20ml are supplied for the cell separation together with instructions as in Example 1.

Instructions to operator

Before measuring:

Switch on the spectrophotometer, set the wavelength to 600 nm. Set to fix wavelength and time record. Calibrate recorder. Separate cells on density gradient as described.

Counting of cells

Depending on cell density add either 0.5 mls or 1.0 mls of PBS to the cell pellet. To small tubes add 0.4 mls PBS, 50 μl cell suspension. Count in haemocytometer. Calculate total number of cells taking the dilution factor into account. Spin down the cell suspension. Remove the PBS, add 0.5 mls cold 0.1% Nonidet P40 to each sample and vortex. Place on ice. Take G6PD standard and place on ice.

Prepare reaction mixture

Defrost all ampoules, making sure that DcPIP and PMS are protected from light as these are very light sensitive. Mix A + B + C + D + 50 mls distilled water. Protect this mixture from light. Place it on a 25°C water bath with spectrophotometer. Make up PMS (E) to 4mls with distilled water. Protect from light.

MEASUREMENT

The decrease in intensity of DcPIP is measured at 600nm. To 2 cuvettes add 2.95mls reaction mixture. Add 50μl of PMS(E) to both. Invert to mix. Add 0.5mls NP40 carefully to one cuvette. Ensure no bubbles are present (invert). Place this into the sample section. Place the other cuvette into the reference section. Add 0.5mls detergent sample into this cuvette, mix with rounded plastic paddle and immediately autozero and measure rate (decrease in absorbance) for 2 mins.

Standard

Repeat as above, but use detergent diluted G6PD enzyme instead.

Our results using this method have shown that samples from normal smears taken from patients visiting a routine health centre have very low G6PD activity compared to cases of CIN. All the cases of CIN were diagnosed by conventional histology. The data below illustrates the potential of this method to produce the minimum number of false positives and false negatives, although it does not distinguish between grades of CIN.

Table 3

| G6PD activity (units/min per $10^4$ cells x $10^{-5}$) | | | | |
|---|---|---|---|---|
| Normal | CIN 1 | CIN 2 | CIN 3 | invasive carcinoma |
| 0 | 0 | 19 | 180 | 6000 |
| 0 | 20 | 30 | 230 | 10,000 |
| 0 | 45 | 46 | 430 | |
| 0 | 53 | 59 | 530 | |
| 0 | 74 | 67 | 550 | |
| 0 | 181 | 110 | 600 | |
| 0 | 250 | 310 | 605 | |
| 0 | 328 | 340 | 620 | |
| 1.4 | 820 | 450 | 803 | |
| 2.0 | | 890 | 917 | |
| 2.0 | | | 946 | |
| 3.0 | | | 2940 | |
| 8.9 | | | | |
| 6.0 | | | | |
| 7.0 | | | | |
| 9.7 | | | | |
| 27.0 | | | | |
| 36.0 | | | | |
| 38.0 | | | | |

For this combined measurement the operator requires the kit formulation given for measurement of G6PD and also a solution of 0.1M hydrogen peroxide, an oxygen electrode, a water bath at 37° C and a chart recorder. If the chamber of the oxygen electrode takes a volume of 1.9 mls the cell lysate will occupy 1.5 mls. 200µl of peroxide (0.1M) are then added and the rate followed after calibration of the monitor and chart recorder.

Assuming that the activities measured are related to the amount of enzyme present, measurement of the amount of G6PD present in cells instead of activity, is possible by the use of a fluorescent antibody to human G6PD which can be estimated by flow cytometry. This has the advantage of very rapid assessment, single cell analysis and the possible omission of the cell separation, because of the facility of flow cytometry to be able to discriminate between cell populations.

EXAMPLE 4 : Enzyme Ratio Method--G6PD/Catalase

Modification of the assays described in the previous Examples includes the simultaneous measurement of a second parameter which is decreased in malignant cells. Enzymes such as xanthine oxidase and catalase are easily measured and detection of abnormality can be improved by expression of increased enzyme and decreased enzyme activities as a ratio. As an example the simultaneous measurement of catalase in separated cells is described.

The cells are processed as described above for the biochemical assay, i.e. gradient separation, cell counting and lysis. The sample is divided and one half of the lysate is used for G6PD determination, the other half of the lysate is used to determine catalase activity. Catalase readily decomposes hydrogen peroxide to oxygen and water:

$$2H_2O_2 \rightarrow 2H_2O + O_2$$

The oxygen produced can be readily monitored by an oxygen electrode. The amount of oxygen produced is expressed as % $O_2$ production per minute per $10^4$ cells x $10^{-5}$. Therefore the ratio of e.g. G6PD to catalase activity can be expressed as:

$$\frac{\text{Rate (G6P)/min/10}^4 \text{ cells x } 10^5}{\text{Rate (catalase)/min/10}^4 \text{cells x } 10^{-5}} = \frac{\text{G6PD activity}}{\text{catalase activity}}$$

Some examples of catalase and simultaneous G6PD estimations are given below:

Table 4

| diagnosis | catalase | G6PD | Ratio |
|---|---|---|---|
| | %$O_2$/min/10$^4$cells 10$^{-5}$ | u/min/10$^4$cells 10$^{-5}$ | G6PD/catalase |
| normal | 17 | 0 | 0 |
| normal | 56 | 0 | 0 |
| normal | 27 | 0 | 0 |
| normal | 30 | 0 | 0 |
| normal | 32 | 0 | 0 |
| normal | 39 | 0 | 0 |
| normal | 59 | 0 | 0 |
| normal | 98 | 0 | 0 |
| normal | 100 | 0 | 0 |
| normal | 233 | 0 | 0 |
| normal | 435 | 0 | 0 |
| normal | 539 | 0 | 0 |
| CIN 1 | 36 | 0 | 0 |
| CIN 1 | 23 | 139 | 6.4 |
| CIN 1 | 36 | 453 | 12.6 |
| CIN 1 | 9 | 471 | 52.3 |
| CIN 2 | 62 | 90 | 1.5 |
| CIN 2 | 11 | 2.3 | 2.1 |
| CIN 2 | 73 | 2023 | 28.0 |
| CIN 2 | 6 | 400 | 67.0 |
| CIN 2 | 0.44 | 163 | 370.0 |
| CIN 3 | 33 | 238 | 7.2 |
| CIN 3 | 36 | 458 | 13.0 |
| CIN 3 | 33 | 440 | 13.3 |
| CIN 3 | 19 | 435 | 23.0 |
| CIN 3 | 9.3 | 500 | 54.0 |
| CIN 3 | 4.7 | 284 | 60.0 |

**Claims**

1. A method of testing for a malignant or pre-malignant condition of the cervix by examination of a cervical smear, which comprises selecting a fraction of the smear consisting predominantly of squamous epithelial cells and examining the selected fraction to determine characteristics indicative of the said malignant or pre-malignant condition.

2. A method according to claim 1, in which separation is effected by density gradient centrifugation.

3. A method according to claim 1 or 2, in which the separated fraction is, or corresponds to, the fraction of density range from about 1.035 to about 1.055 g/ml as measured in a Percoll density gradient.

4. A method according to claim 2 or 3, wherein the separated cells are further purified by gradient centrifugation.

5. A method according to any of the preceding claims, in which the test is performed on intact cells.

6. A method according to claim 5, which comprises the quantitative estimation of a marker formed in or taken up by the cells.

7. A method according to any of claims 1 to 4, in which the test is performed on lysed cells.

8. A method according to any of the preceding claims, in which the test is for an abnormal level of an enzyme associated with proliferating cells.

9. A method according to claim 8, in which the enzyme tested for is one selected from the group consisting of glucose-6-phosphate dehydrogenase, 6-phosphogluconate dehydrogenase, ornithine decarboxylase, thymidine kinase, and ribonucleotide reductase.

10. A method according to claim 8 or 9, which comprises also measuring the level of an enzyme which is repressed in the condition tested for and determining the ratio of the levels of the two respective enzymes.

11. A method according to claim 10, in which the repressed enzyme is catalase or xanthine oxidase.

12. A method according to any of claims 1 to 4, which comprises subjecting the smear to discontinuous gradient centrifugation, harvesting cells corresponding to combined fractions of cells having densities within the range 1.035 to 1.055 g/ml as measured in a Percoll gradient, counting and then lysing the harvested cells, determining the amounts in the lysate of two enzymes one of which is enhanced and the other repressed in the condition under test, and calculating the ratio of the amounts of the two enzymes.

13. A reagent kit for use in the detection of a pre-malignant or malignant condition of the cervix by testing a cervical smear, said kit comprising reagent material for separating squamous epithelial cells from the smear, a substrate for an enzyme which is present at an increased level in the condition tested for, a cycling electron acceptor for the enzyme, and a final electron acceptor which upon reduction provides a measurable indication which correlates with the level of said enzyme present in the smear.

14. A reagent kit according to claim 13, comprising separate solutions of gradient materials to provide a suitable density range for a discontinuous gradient, or dry materials for reconstitution as such solutions.

15. A reagent kit according to claim 14, comprising materials for a Percoll gradient over the density range 1.035 to 1.055 g/ml or other gradient materials equivalent thereto.

16. A reagent kit according to any of claims 13 to 15, in which the enzyme is selected from the group consisting of glucose-6-phosphate dehydrogenase, 6-phosphogluconate dehydrogerase, ornithine decarboxylase, thymidine kinase, and ribonucleotide reductase.

17. A reagent kit according to any of claims 13 to 16, comprising a reagent for lysing the epithelial cells.

18. A reagent kit according to any of claims 13 to 17, comprising one or more reagents for estimating the level of an enzyme which is repressed in the condition tested for.

19. A reagent kit according to claim 18, in which the repressed enzyme is catalase.

20. A reagent kit according to any of claims 13 to 19, in which the cycling electron acceptor is phenazine methosulphate.

**Patentansprüche**

1. Verfahren zum Testen eines malignen oder prämalignen Zustandes des Gebärmutterhalses durch Untersuchung eines Zervikalabstriches, welches die Auswahl einer vorherrschend aus schuppigen Epithelzellen bestehenden Fraktion des Abstriches und die Untersuchung der ausgewählten Fraktion zur Bestimmung von Eigenarten aufweist, die für den erwähnten malignen oder prämalignen Zustand kennzeichnend sind.

2. Verfahren nach Anspruch 1, bei dem die Trennung durch Dichtegradientenzentrifugation bewirkt wird.

3. Verfahren nach Anspruch 1 oder 2, bei dem die abgetrennte Fraktion die Fraktion des Dichtebereiches zwischen etwa 1,035 und etwa 1,055 g/ml, gemessen als Percoll-Dichtegradient, ist oder dieser entspricht.

4. Verfahren nach Anspruch 2 oder 3, bei dem die abgetrennten Zellen weiter durch Gradientenzentrifugation gereinigt werden.

5. Verfahren nach einem der vorangehenden Ansprüche, bei dem der Test an intakten Zellen ausgeführt wird.

6. Verfahren nach Anspruch 5, welches die quantitative Bestimmung eines in den Zellen gebildeten oder durch diese aufgenommenen Markers aufweist.

7. Verfahren nach einem der Ansprüche 1 bis 4, bei dem der Test an lysierten Zellen ausgeführt wird.

8. Verfahren nach einem der vorangehenden Ansprüche, bei dem der Test auf einen anormalen Pegel eines mit wuchernden Zellen verknüpften Enzyms gerichtet ist.

9. Verfahren nach Anspruch 8, bei dem das getestete Enzym aus der aus Glukose-6-Phosphat-Dehydrogenase, 6-Phosphoglukonat-Dehydrogenase, Ornithin-Dekarboxylase, Thymidin-Kinase und Ribonucleotid-Reduktase bestehenden Gruppe ausgewählt wird.

10. Verfahren nach Anspruch 8 oder 9, welches auch das Messen des Pegels des Enzyms, das im zu testenden Zustand reprimiert ist, und das Bestimmen des Verhältnisses der Pegel der beiden entsprechenden Enzyme aufweist.

11. Verfahren nach Anspruch 10, wobei das reprimierte Enzym Katalase oder Xanthin-Oxidase ist.

12. Verfahren nach einem der Ansprüche 1 bis 4, welches das Unterziehen des Abstriches einer diskontinuierlichen Gradientenzentrifugation, das Gewinnen der Zellen entsprechend kombinierter Fraktionen von Zellen mit Dichten innerhalb des Bereiches von 1,035 bis 1,055 g/ml, gemessen als Percoll-Gradient, das Zählen und anschließende Lysieren der gewonnenen Zellen, das Bestimmen der Anteile der beiden Enzyme im Lysat, von denen das eine in dem getesteten Zustand verstärkt und das andere reprimiert ist, und das Berechnen des Verhältnisses der Anteile der beiden Enzyme aufweist.

13. Reagens-Kit zur Verwendung beim Nachweis eines prämalignen oder malignen Zustandes des Gebärmutterhalses durch Testung eines Zervikalabstrichs, wobei das Kit ein Reagenzmittel zur Abtrennung schuppiger Epitelzellen aus dem Abstrich, ein Substrat für ein Enzym, welches in dem getesteten Zustand auf erhöhtem Pegel vorhanden ist, einen zyklierenden Elektronenakzeptor für das Enzym und einen finalen Elektronenakzeptor aufweist, welcher durch Reduktion eine meßbare Anzeige ergibt, welche mit dem Pegel des in dem Abstrich vorhandenen Enzyms korreliert.

14. Reagens-Kit nach Anspruch 13, welches getrennte Lösungen von Gradientenmaterialien zur Erreichung eines geeigneten Dichtebereiches für einen diskontinuierlichen Gradienten oder Trockenmaterialien zur Neuherstellung solcher Lösungen aufweist.

15. Reagens-Kit nach Anspruch 14, welches Materialien für einen Percoll-Gradienten über den Dichtebereich von 1,035 - 1,055 g/ml oder andere hierzu äquivalente Gradientenmaterialien aufweist.

16. Reagens-Kit nach einem der Ansprüche 13 bis 15, bei dem das Enzym aus der aus Glukose-6-Phosphat-Dehydrogenase, 6-Phosphoglukonat-Dehydrogenase, Ornithin-Dekarboxylase, Thymidin-Kinase und Ribonucleotid-Reduktase bestehenden Gruppe ausgewählt ist.

17. Reagens-Kit nach einem der Ansprüche 13 bis 16, welches ein Reagens zum Lysieren der Epitelzellen aufweist.

18. Reagens-Kit nach einem der Ansprüche 13 bis 17, welches eines oder mehrere Reagenzien zur annähernden

# EP 0 641 437 B1

Bestimmung des Pegels eines Enzyms aufweist, welches im getesteten Zustand reprimiert ist.

**19.** Reagens-Kit nach Anspruch 18, bei dem das reprimierte Enzym Katalase ist.

**20.** Reagens-Kit nach einem der Ansprüche 13 bis 19, bei dem der zyklierende Elektronenakzeptor Phenazin-Methosulfat ist.

**Revendications**

**1.** Méthode pour rechercher une affection cancéreuse ou précancéreuse du col de l'utérus par l'examen de la glaire cervicale, comprenant la sélection d'une fraction de glaire comprenant de façon prédominante des cellules de l'épithélium pavimenteux et l'examen de la fraction sélectionnée pour déterminer des caractéristiques de mise en évidence de ladite affection cancéreuse ou précancéreuse.

**2.** Méthode selon la revendication 1, dans laquelle la séparation est effectuée par centrifugation en gradient de densité.

**3.** Méthode selon la revendication 1 ou 2, dans laquelle la fraction séparée est, ou correspond à, la fraction dont la densité, mesurée dans un gradient de densité de Percoll, se situe dans l'intervalle d'environ 1,035 à environ 1,055 g/ml.

**4.** Méthode selon la revendication 2 ou 3, dans laquelle les cellules séparées sont encore purifiées par une centrifugation en gradient.

**5.** Méthode selon une quelconque des revendications précédentes, dans laquelle le test est réalisé sur des cellules intactes.

**6.** Méthode selon la revendication 5, qui comprend l'estimation quantitative d'un marqueur formé dans les cellules ou capturé par les cellules.

**7.** Méthode selon une quelconque des revendications 1 à 4, dans laquelle le test est réalisé sur des cellules lysées.

**8.** Méthode selon une quelconque des revendications précédentes, dans laquelle le test concerne un taux anormal d'une enzyme associée aux cellules en prolifération.

**9.** Méthode selon la revendication 8, dans laquelle l'enzyme impliquée dans le test est une enzyme choisie dans le groupe constitué par la glucose-6-phosphate déshydrogénase, la 6-phosphogluconate déshydrogénase, l'ornithine décarboxylase, la thymidine kinase et la ribonucléotide réductase.

**10.** Méthode selon la revendication 8 ou 9, comprenant aussi la mesure du taux d'une enzyme réprimée dans les conditions du test et la détermination du rapport des taux des deux enzymes respectifs.

**11.** Méthode selon la revendication 10, dans laquelle l'enzyme réprimée est la catalase ou la xanthine oxydase.

**12.** Méthode selon une quelconque des revendications 1 à 4, comprenant le traitement de la glaire par une centrifugation en gradient discontinu, le recueil des cellules correspondant à l'ensemble des fractions de cellules dont les densités, mesurées dans un gradient de Percoll, se situent dans l'intervalle d'environ 1,035 à environ 1,055 g/ml, la numération et ensuite la lyse des cellules recueillies, la détermination des quantités, dans le lysat, de deux enzymes dont l'une est augmentée et l'autre réprimée dans les conditions du test et le calcul du rapport des quantités des deux enzymes.

**13.** Kit de réactifs à utiliser dans la détection d'une affection cancéreuse ou précancéreuse du col de l'utérus par le test de la glaire cervicale, ledit kit comprenant un réactif pour la séparation des cellules de l'épithélium pavimenteux de la glaire, un substrat pour une enzyme présente à un taux augmenté dans les conditions du test, un accepteur pour l'électron du cycle pour l'enzyme et un accepteur de l'électron final qui, par réduction, donne une indication mesurable, en corrélation avec le taux de ladite enzyme présente dans la glaire.

14. Kit de réactifs selon la revendication 13, comprenant des solutions séparées de matières pour gradient pour obtenir un intervalle de densité approprié pour un gradient discontinu ou des matières sèches pour la reconstitution de telles solutions.

15. Kit de réactifs selon la revendication 14, comprenant des matières pour un gradient de Percoll pour des densités dans l'intervalle de 1,035 à 1,055 g/ml ou d'autres matières pour gradients équivalents.

16. Kit de réactifs selon une quelconque des revendications 13 à 15, dans lequel l'enzyme est choisie dans le groupe constitué par la glucose-6-phosphate déshydrogénase, la 6-phosphogluconate déshydrogénase, l'ornithine décarboxylase, la thymidine kinase et la ribonucléotide réductase.

17. Kit de réactifs selon une quelconque des revendications 13 à 16, comprenant un réactif pour la lyse des cellules épithéliales.

18. Kit de réactifs selon une quelconque des revendications 13 à 17, comprenant un ou plusieurs réactifs pour estimer le taux d'une enzyme réprimée dans les conditions du test.

19. Kit de réactifs selon la revendication 18, dans lequel l'enzyme réprimée est la catalase.

20. Kit de réactifs selon une quelconque des revendications 13 à 19, dans lequel l'accepteur de l'électron du cycle est le méthosulfate de phénazine.